# EUROPEAN PATENT APPLICATION

(11) **EP 2 412 374 A1**
(43) Date of publication of application: **01.02.2012**
(21) Application number: 09842242.1
(22) Date of filing: 26.03.2009
(51) Int. Cl.: A61K 31/702, A23L 1/29, A61K 31/715

(54) **PHARMACEUTICAL COMPOSITION FOR ENHANCING ADIPONECTIN PRODUCTION AND FOOD USEFUL THEREFOR**

(71) Applicant: Ajinomoto General Foods, Inc., Shinjuku-ku Tokyo 163-1440 (JP); Kraft Foods Global Brands LLC, Northfield, IL 60093 (US)
(72) Inventor: TSUCHIYA, Tomohiro, Tokyo 169-0073 (JP); HOSHINO, Izumi, Tokyo 169-0073 (JP); KUMAOH, Toshio, Tokyo 169-0073 (JP); OZAKI, Kazuto, Tokyo 169-0073 (JP); FUJII, Shigeyoshi, Tokyo 169-0073 (JP)
(74) Representative: Setna, Rohan P.
(86) International application number: PCT/JP2009/056118
(87) International publication number: WO 2010/109628

(57) **Abstract**

Disclosed are a pharmaceutical product having excellent adiponectin production ability, and food useful for the pharmaceutical product. The pharmaceutical product contains, as active ingredients, a sugar having a polymerisation degree of 2-10, wherein mannose units account for not less than 50% based on the number, and a chlorogenic acid.

## Description

### Technical field

The present invention relates to a pharmaceutical composition for enhancing adiponectin production, which contains chlorogenic acid and a sugar having a degree of polymerization between 2 and 10 wherein mannose units account for at least 50% of the number of units, as well as a food useful therefor.

### Prior art

Metabolic syndrome associated with diabetes, lipid metabolism anomalies, and hypertension and caused by accumulation of visceral fat has become a serious problem. Accumulation of visceral fat is caused by insufficient daily exercise, a Western diet that is high in fats, particularly animals fats, the overeating associated with stress, and the like, and is accompanied by an increase in adipocyte size. Many biologically active substances (adipocytokines) are excreted from these adipocytes. One of these substance is adiponectin, which is referred to as a "good cytokine" when secreted in abundance in reverse correlation to the size of adipocytes. Adiponectin is called a "good cytokine" because it has the function of inhibiting metabolic syndrome in opposition to the cause of this secretion.

It has been ascertained that hypoadiponectinemia, which is a reduction in the blood concentration of adiponectin corresponding to this function of adiponectin, is related to insulin resistance, hypotension, high triglyceride levels, artherosclerotic cardiovascular disease, lipid metabolism anomalies, etc. Furthermore, it is reported that the incidence of metabolic syndrome is even high among people who are not obese but merely have a genetic disposition toward hypoadiponectinemia.

Consequently, enhancing the production of adiponectin promises to inhibit the development of metabolic syndrome and prevent or improve lifestyle-associated illness.

JP (Kokai) 2006-193502 and JP (Kokai) 2007-246443 disclose several food components that enhance adiponectin production. JP (Kokai) 2006-193502 discloses that apple extract containing a chlorogenic acid raises adiponectin levels. It is reported in Diabetes Research and Clinical Practice (2004 Sep; 65(3):227-34) that the caffeine contained in coffee and tea is capable of producing adiponectin, but this effect is not large.

The residue of coffee extract was previously incinerated or treated as an industrial waste product. In recent years, the residue of coffee extract has been used as compost starting material or activated charcoal starting material, but these have not been useful methods for employing underused resources and there is a desire to establish a method for further use of the residue of coffee extract.

The inventors previously reported that mannooligosaccharide purified from the residue of coffee extract has excellent properties in terms of reducing body fat and improving serum lipids (JP (Kokai) 2006-082137, JP (Kokai) 2006-169256). Moreover, Yakuri to Chiryo (2007, 35 1107-1114) reports that there is a tendency toward improvement in the serum adiponectin level with singular administration of a mannooligosaccharide.
Patent Reference 1: JP (Kokai) 2006-193502
Patent Reference 2: JP (Kokai) 2007-246443
Patent Reference 3: JP (Kokai) 2006-082137
Patent Reference 4: JP (Kokai) 2006-169256
Non-Patent Reference 1: Diabetes Research and Clinical Practice, 2004 Sep; 65(3):227-34.
Non-Patent Reference 2: Yakuri to Chiryo 2007 35 1107-1114

### Disclosure of invention

### Problems to be solved by invention

An object of the present invention is to economically and simply provide a pharmaceutical drug having excellent adiponectin-producing capability and a food that can be easily used in daily life.

### Means for Solving Problems

The inventors performed intense studies in order to solve such a problem and discovered that a chlorogenic acid and a sugar having a degree of polymerization between 2 and 10 wherein mannose units account for at least 50% of the number of units are obtained from a mannan-rich food material, particularly a hydrolysis product of the residue of coffee extract, and that these have a synergistic effect in terms of enhancing adiponectin production.

Consequently, the present invention is a pharmaceutical composition for enhancing adiponectin production, which contains a chlorogenic acid and a sugar having a degree of polymerization between 2 and 10 wherein mannose units account for at least 50% of the number of units. Preferably this sugar has a degree of polymerization between 2 to 10 wherein mannose units account for at least 90%, further preferably at least 95%, of the number of units. It is most preferred that the sugar has a degree of polymerization of 2 to 10 and is formed from mannose units alone.

In a preferred embodiment the sugar is β-1, 4-mannooligosaccharide.

In a preferred embodiment, the sugar is obtained by hydrolysis of mannan, and preferably this mannan is obtained from coffee beans and/or the residue of coffee extract.

The present invention is also a method for enhancing adiponectin production, and is further a method for administering the composition according to claim 1, in the effective dose, to a patient requiring enhancement of adiponectin production.

The present invention also is a food containing a chlorogenic acid and a sugar having a degree of polymerization between 2 and 10 wherein mannose units account for at least 50% of the number of units.

### Effect of invention

The pharmaceutical composition of the present invention enhances the production of adiponectin *in vivo.* Moreover, the food of the present invention can be taken with meals every day and plays a role in the production of adiponectin *in vivo.* Furthermore, the components of the composition of the present invention can be obtained from a waste product such as the residue of coffee extract and, therefore, the present invention also is effective in promoting the efficient use of a waste product.

### Brief description of drawings

[Figure 1] shows the synergistic effect obtained in Working Example 3 when the oligosaccharide of Working Example 1 + the chlorogenic acid of Working Example 2 are administered.
[Figure 2] shows the reducing effect of liquid coffee on abdominal fat surface area.

### Embodiments of invention

The structural units of the sugar having a degree of polymerization between 2 and 10 wherein mannose units account for at least 50% of the number of units are, other than mannose, at least one monosaccharide selected from glucose, fructose and galactose.

The sugar having a degree of polymerization between 2 and 10 wherein mannose accounts for at least 50% of the number of units can be produced by hydrolysis of mannan. The mannan to be hydrolyzed can be obtained from several sources. Examples are coffee beans, coffee extract residue, copra meal obtained from coconuts, the mannan contained in Huacra palm, tsukune potato mannan and Japanese yam mannan. Mannan obtained from coffee beans and/or the residue of coffee extract is preferred. When ground unroasted coffee is subjected to extraction using a commercial extractor, the galactose that is the side chain of the galactomannan contained in the unroasted coffee is solubilized at this time and the arabinogalactan is solubilized by hydrolysis. Consequently, the coffee extract residue is rich in mannan and is estimated to take on a linear structure. On the other hand, cellulose remains as a residue that is difficult to decompose, but the conditions under which mannan is specifically hydrolyzed without decomposing the cellulose can be selected as needed.

The mannan obtained in this way can be treated by one or two or more methods selected from acid hydrolysis, high-temperature hydrolysis, enzyme hydrolysis, and microorganism fermentation. Preferably, a sugar mixture having a degree of polymerization between 2 and 10 wherein mannose units account for at least 50% of the number of units is obtained when bleaching and deodorization are performed by bone ash treatment, activated carbon treatment, carbon dioxide saturation, resin absorption, the magnesia method, or solvent extraction and the product is purified by desalination and deacidification by ion-exchange resin treatment, ion-exchange membrane treatment, electro-osmosis, etc. The sugar having a degree of polymerization between 2 and 10 wherein mannose units account for at least 50% of the number of mannose units of the present invention can also be produced by subjecting the glucomannan contained in konjaku potato, lily, narcissus, or red spider lily, or the galactomannan contained in locust bean gum or guar gum, to the above-mentioned treatment and purification in order to increase the mannose percentage. Consequently, what is simply referred to as "mannan" in the present invention includes the mannan that is a polysaccharide whose structural units are D-mannose, as well as the galactomannan and glucomannan that are polysaccharides whose structural units are mannose and galactose or glucose. It should be noted that D-mannose is aldohexose and the configuration of the hydroxyl groups bonded to the carbon adjacent to the carboxyl groups in the D-glucose is reversed. Examples of acids used for acid hydrolysis are sulfuric acid and hydrochloric acid. Acidity is a pH between 0 and 2.

The high-temperature hydrolysis temperature is 190 to 250 °C.

Enzyme hydrolysis is performed by suspending mannan or a source of mannan in an aqueous solvent and then adding commercial cellulase or hemicellulase and stirring. The concentration of the oxygen used is 0.01% to 1% and the temperature is 30 to 80°C.

Microorganism fermentation is performed by inoculating mannan or a mannan source suspended in an aqueous medium with a microorganism that produces cellulase or hemicellulase and then culturing the product. Examples of the bacterial strains are *Aspergillus niger, Bacillus subtilis, and Rhizopus oryzae.*

A styrene divinyl benzene polymer is an example of the resin used for resin adsorption. SK-1B is an example of an ion-exchange resin used for ion-exchange resin treatment.

The reaction solution containing mannooligosaccharide having a degree of polymerization between 2 and 10 obtained by the above-mentioned method can be purified as needed. Examples of purification methods are bone ash treatment, activated carbon treatment, carbon dioxide saturation, resin absorption, the magnesia method, and solvent extraction for bleaching and deodorization and ion-exchange resin treatment, ion-exchange membrane treatment, or electro-osmosis for desalination and deacidification. The combination of purification methods and purification conditions can be selected as needed in accordance with the color, amount of salt and acid, and the like in the reaction solution containing mannooligosaccharide having a degree of polymerization between 2 and 10.

Methods for acid and/or high-temperature hydrolysis of the residue of a coffee extract in particular are disclosed in JP (Kokai) 61-96947 and JP (Kokai) 2-200147. Hydrolysis can be performed by adding an acid catalyst to the residue of used coffee extract produced by a commercial coffee multistep extraction system, or mannan can be obtained by hydrolysis for a short amount of time under a high temperature without adding an acid catalyst. Although the use of a tube-shaped plug-flow reactor is preferred, good results will be obtained regardless of the reactor used as long as the reaction is for a short period of time under a relatively high temperature. It is possible to adjust the reaction time and reaction temperature, decompose the mannan having a degree of polymerization between 10 and 40 to a sugar having a degree of polymerization between 2 and 10 by performing solubilization and hydrolysis, and then separate the sugar from the coffee extract residue. There are no special restrictions to the type of coffee bean used or the region where it is produced. Arabica, robusta, and liberica coffee beans produced in Brazil and Columbia can be used. It is possible to use one type of coffee bean alone, or a blend of two or more beans can be used. Usually, it is also possible to use coffee beans of poor quality or small coffee beans that would normally be discarded because they have no commercial value. Very light roasted, light roasted, medium roasted, and dark roasted coffee beans roasted by a conventional roaster (direct flame, hot air, infrared, charcoal fire), and the ground roasted coffee (including a variety of grinds, such as coarse grind, medium coarse grind, medium grind, medium fine grind, fine grind) obtained by grinding roasted coffee beans using a conventional grinder or roll mill can be used.

Moreover, the residue of coffee extract can be the residue of coffee extract obtained from any source and by any method under normal pressure or increased pressure as long as it is the product of extraction treatment of a ground roasted coffee during the production of conventional liquid coffee or instant coffee.

The "chlorogenic acid" that is present with the sugar having a degree of polymerization between 2 and 10 wherein mannose units account for at least 50% of the number of units includes chlorogenic acid, as well as chlorogenic acid derivatives, such as cryptochlorogenic acid, neochlorogenic acid, and isochlorogenic acid. The chlorogenic acid here is 5-caffeoylquinic acid wherein the caffeic acid has formed an ester bond with the hydroxyl group at position 5 of quinic acid, the cryptochlorogenic acid is 4-caffeoylquinic acid wherein caffeic acid has formed an ester bond with the hydroxyl group at position 4 of quinic acid, the neochlorogenic acid is 3-caffeoylquinic acid wherein caffeic acid has formed an ester bond with the hydroxyl group at position 3 of quinic acid, and the iosochlorogenic acid is caffeoylquinic acid wherein caffeic acid has formed an ester bond with two of the hydroxyl groups at positions 3, 4, and 5 of quinic acid (such as 3,4-dicaffeoylquinic acid, 3,5-dicaffeoylquinic acid, or 4,5-dicaffeoylquinic acid). Other examples of chlorogenic acids are feruloylquinic acid wherein ferulic acid has formed an ester bond with one of the hydroxyl groups at positions 3, 4, and 5 of quinic acid (such as 5-feruloylquinic acid), and feruloylcafeoylquinic acid wherein caffeic acid and ferulic acid have formed ester bonds with two of the hydroxyl groups at positions 3, 4, and 5 of quinic acid (for instance, 3-feruloyl-4-caffeoylquinic acid). Other derivatives are biologically acceptable derivatives, such as salt and sugar esters of chlorogenic acids. The salt can be an alkali metal salt such as sodium or potassium or an alkaline earth metal salt such as calcium or magnesium. The "chlorogenic acid" can be one of these acids, or a mixture of multiple acids.

Chlorogenic acids can be obtained by extraction from synthetic or natural substances, but one extracted from a plant is preferred. The plant can be Ku Ding tea, yerba mate tea, macropoda holly, black mustard, *Ilex latifolia,* fresh coffee beans, or nandina leaves, or basil, common mugwort, *Petasites japonica,* solanum, *Perilla frutescens,* sunflower, common mugwort, or sweet potato. The preferred chlorogenic acid is a mixture of chlorogenic acids extracted, particularly after decaffeination, from fresh coffee beans that are seeds of coffee belonging to the family *Rubiaceae* (arabica, robusta).

The extraction of chlorogenic acids is performed using an aqueous solution of L-ascorbic acid or citric acid, hot water, or an aqueous solution containing ethanol.

The pharmaceutical composition of the present invention is obtained by formulating a mixture of a sugar having a degree of polymerization between 2 and 10 wherein mannose units account for at least 50% of the number of units, a chlorogenic acid, and other excipients and additives into a tablet, powder, granule, capsule, gel capsule, dry syrup, troche, or another form.

Examples of excipients and additives are solids such as lactose, sucrose, crystalline cellulose, corn starch, agar, pectin, stearic acid, magnesium stearate, lecithin, and sodium chloride, and liquids such as glycerin, polyvinylpyrrolidone, olive oil, ethanol, benzyl alcohol, propylene glycol, and water. The amount of sugar having a degree of polymerization between 2 and 10 wherein mannose units account for at least 50% of the number of units is 0.1 to 90 wt%, preferably 1 to 70 wt%, more preferably 5 to 50 wt%, of the total amount of pharmaceutical composition, and the amount of chlorogenic acid is 0.01 to 20 wt%, preferably 0.1 to 15 wt%, further preferably 0.5 to 10 wt%, of the total amount of pharmaceutical composition. Known adiponectin potentiators such as caffeine can be added as needed.

The pharmaceutical composition of the present invention displays adiponectin production-enhancing effects particularly when taken orally.

When the pharmaceutical composition of the present invention is orally administered to a human, the dose varies with age, weight, sex, and the method of administration, but the daily dose in an adult male is preferably approximately 10 to 500 mg/kg body weight, more preferably approximately 30 to 70 mg/kg body weight.

Examples of foods containing a chlorogenic acid and a sugar having a degree of polymerization between 2 and 10 wherein mannose units account for at least 50% of the number of units are liquid coffee, instant coffee, and coffee mix beverages. The term liquid coffee means coffee and coffee-containing beverages that are sold in cans or so-called PET bottles, and the term instant coffee means soluble powdered coffee obtained by using spray or freeze drying to remove the water content from extract obtained by extracting ground roasted coffee with hot water. Moreover, examples of coffee mix beverages are beverages obtained by adding table sugar, powdered creamer, and the like to a soluble powdered coffee. The chlorogenic acid and sugar having a degree of polymerization between 2 and 10 wherein mannose units account for at least 50% of the number of units can be purified by bleaching, deodorization, deacidification, and the like with activated charcoal, an ion exchange resin, a solvent, and the like in order to provide a coffee that is richer in coffee flavor and fragrance. The amount of sugar having a degree of polymerization between 2 and 10 wherein mannose units account for at least 50% of the number of units is 0.1 to 90 wt%, preferably 0.5 to 80 wt%, more preferably 1 to 67 wt%, of the total amount of food, and the amount of chlorogenic acid is 0.001 to 30 wt%, preferably 0.01 to 20 wt%, more preferably 0.01 to 10 wt%.

The daily amount of food to be taken is such that the amount of sugar having a degree of polymerization between 2 and 10 wherein mannose units account for at least 50% of the number of units is preferably 0.5 to 20 g, more preferably 1.0 to 10 g, and the amount of chlorogenic acid is preferably 10 to 1,000 mg, more preferably 50 to 250 mg.

The combination of a chlorogenic acid and a sugar having a degree of polymerization between 2 and 10 wherein mannose units account for at least 50% of the number of units can be used in a wide variety of other fields such as cosmetics and feed.

### Examples

The pharmaceutical composition for enhancing adiponectin production of the present invention was prepared and the effects thereof were studied as follows. These examples are specific descriptions of embodiments of the present invention and are not intended to restrict the scope of the present invention.

"DP" is used to represent the degree of polymerization of the sugar in the following examples. It is an abbreviation for the degree of polymerization, and is defined as the number of monosaccharides forming an oligosaccharide. Consequently, a monosaccharide is represented as "DP1," and an oligosaccharide formed from four monosaccharides is represented as "DP4."

### Example 1

### Preparation of sugar having degree of polymerization between 2 and 10 wherein mannose units account for at least 50% of the number of units

10 kg of ground roasted coffee obtained by normal methods was extracted and concentrated using a commercial percolation system to obtain 7 kg of coffee extract residue (dry weight).

This was transferred to a 4 m thermal plug-flow reactor after being ground to a grain size of approximately 1 mm. Then water was added to produce a slurry having a total solid concentration of approximately 14 wt%. The product was pumped to a plug-flow reactor together with high-pressure steam at a rate corresponding to a retention time of 8 minutes and maintained at approximately 210°C using an orifice having a diameter of 6.35 mm. Then the product was sprayed under atmospheric pressure to stop the reaction. The resulting slurry was filtered to obtain a liquid containing a soluble solid content. This liquid was bleached using activated carbon and an adsorbing resin, further desalinated using an ion-exchange resin (Daiyaion SK-1B and Daiyaion SA-12A), concentrated and dried to obtain a mixture of a sugar having a degree of polymerization between 2 and 10 wherein mannose units account for at least 50% of the number of units.

The DP distribution of the oligosaccharide contained in the mixture obtained in this way was 26.6% of DP2, 20.2% of DP3, 17.8% of DP4, 10.9% of DP5, 8.9% of DP6, 6.0% of DP7, 3.6% of DP8, 1.9% of DP9, and 1.7% of DP10. The mannose residue content in the sugar ring was 90% in terms of the number of residues. The bonding pattern of the oligosaccharide was β-1, 4 bonding.

### Example 2

### Preparation of chlorogenic acid

1 kg of Columbian coffee beans that had been 97% decaffeinated by the so-called Swiss water method (EP008398, A. Fischer et al, 1979) were introduced to a 10 L Erlenmeyer flask, 5 L of an aqueous 50 wt% hot ethanol solution heated to 80°C was added, extraction was performed for 3 hours while refluxing, and the resulting extract was concentrated approximately 500% by volume while heating to 70°C using a rotary-type reduced pressure evaporator to obtain 3 L of a concentrate containing fresh coffee bean extract.

This was dried for 24 hours using a vacuum dryer to obtain 0.28 kg of dry chlorogenic acid crude extraction solid content. The results of high-pressure liquid chromatography showed that the active component percentage of the dry product was 99% or more of chlorogenic acid and 0.6% or less of caffeine, with the chlorogenic acid being the primary component.

### Example 3

### Effect of administration of high-fat feed on adiponectin production capability in mice

### Laboratory animals and rearing conditions

ICR female mice were used in the experiment. The mice were reared for one week, which served as a quarantine and an acclimation period, and mice in which no changes in body weight or anomalies in general condition were observed were submitted to the experiment. Rearing was performed under controlled temperature and humidity with the lighting cycle being 12 hours each of light and dark. The feed during the quarantine and acclimation period was solid feed. The animals were allowed to take *ad libitum* CRF-1 (Oriental Kobo Co., Ltd), and tap water. After the preliminary rearing period, the animals were assigned at 10 mice per group such that the average body weight would be approximately equal. The group structure was four groups, a control group, an Example 1 oligosaccharide group, an Example 2 chlorogenic acid group and an Example 1 oligosaccharide + Example 2 chlorogenic acid group.

The high-fat feed was 40% beef tallow feed (Research Diet Co., Ltd.). The control group was started on gavage oral administration of 10 mL/kg body weight of water for injection alone, and the Example 1 oligosaccharide group was started on gavage oral administration of the oligosaccharide in Example 1 dissolved to 3,000 mg/kg body weight in 10 mL/kg body weight of water for injection (with that day being day 0 of administration), and thereafter the feed was changed to high-fat feed. The Example 2 chlorogenic acid group was given the chlorogenic acid of Example 2 dissolved to 100 mg/kg body weight, and the Example 1 oligosaccharide + Example 2 chlorogenic acid group was given each substance mixed and dissolved in the same amount. Thereafter, gavage oral administration of the treatment substance or water for injection was performed once a day every day. Serum was taken 56 days after starting administration and the serum adiponectin concentration was determined by the Western blot method.

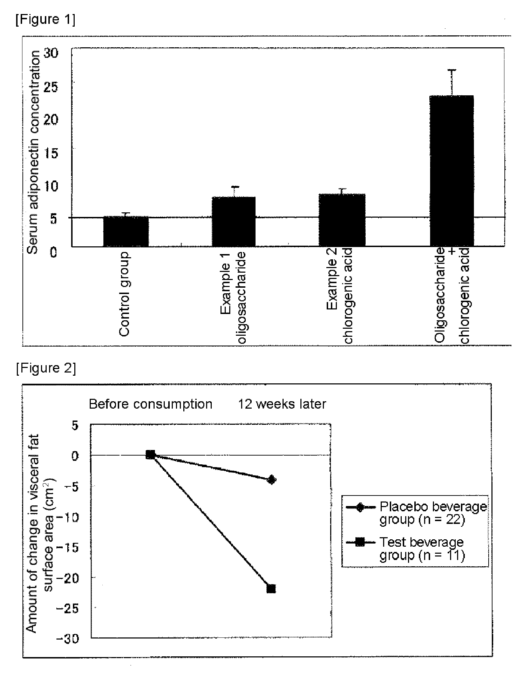

The serum adiponectin concentration (density) of each group is shown in Table 1 and Figure 1 as the average ± SE.

**[Table 1]**

| | Control group | Example 1 oligosaccharide | Example 2 chlorogenic acid | Oligosaccharide + chlorogenic acid |
|---|---|---|---|---|
| Average | 4.47 | 7.56 | 7.95 | 22.73 |
| SE | 0.76 | 1.55 | 1.01 | 4.09 |

When compared to the control group, each of the groups showed a tendency toward a rise in the adiponectin concentration. When compared by the difference obtained by subtracting the average concentration of the control group from the average adiponectin concentration of each group, the group given the Example 1 oligosaccharide and Example 2 chlorogenic acid obviously had an increase in concentration that exceeded the value obtained by simply combining the dose of the two groups, indicating that the effect was synergistic. Based on these findings, it was considered that the production of adiponectin is synergistically enhanced by the combined administration of a chlorogenic acid and a sugar having a degree of polymerization between 2 and 10 wherein mannose units account for at least 50% of the number of units.

### Example 4

### Preparation of liquid coffee for human testing

Milk component, table sugar, dextrin, and artificial sweetener were added to coffee extract liquid, the mixture was sterilized by conventional methods, and the sterile product was filled into 275 g bottles to prepare the placebo beverage. The coffee extract liquid contained chlorogenic acid. Mannooligosaccharide and chlorogenic acid were added in place of the dextrin to prepare a sample beverage by the same method as used for the placebo beverage. The composition% of each test sample and the content per 275 g are shown in Table 2.

**[Table 2]**

| Composition% | | Placebo beverage | Test sample |
|---|---|---|---|
| | Coffee solid content | 0.9 | 0.9 |
| | Table sugar | 2.0 | 2.0 |
| | Milk | 7.1 | 7.1 |
| | Dextrin | 2.2 | 0 |
| | Mannooligosaccharide | 0 | 3.0 |
| | Artificial sweetener | 0.1 or less | 0.1 or less |
| | Water | 88 | 87 |
| | | | (%) |
| Component table | Energy | 79 kcal | 78 kcal |
| | Lipids | 1.1 g | 1.1 g |
| | Mannooligosaccharide | - | 3.0 g |
| | Chlorogenic acid | 90 mg | 160 mg |

### Effect of liquid coffee in terms of promoting adiponectin production

Adult men and women age 30 or younger having a BMI of 25 or greater consumed 275 g per day of each test beverage for 12 consecutive weeks and the adiponectin concentrations of serum sampled before and after the test were compared. The adiponectin concentration was determined by ELISA.

As shown in Table 3, the placebo beverage group showed a slight reduction in the adiponectin concentration after 12 weeks. On the other hand, the mannooligosaccharide + additional chlorogenic acid test beverage group showed a rise in the adiponectin concentration.

**[Table 31**

| | Placebo beverage group (n = 22) | Test beverage group (n = 11) |
|---|---|---|
| Before consumption | 9.2 | 10.7 |
| 12 weeks later | 8.8 | 11.5 |
| (µg/mL) | | |

### Reducing effect on abdominal fat surface area

A CT image was obtained of the abdominal fat before consumption and 12 weeks later and the amount of change in the surface area was compared. As shown in Table 4 and Figure 2, in contrast to the fact that virtually no change was seen in the placebo beverage group, there was an obvious reduction in the mannooligosaccharide + additional chlorogenic acid test beverage group. The rise in the adiponectin concentration was related to the reduction in the amount of fat, indicating that adiponectin plays a role in improving metabolic syndrome.

**[Table 41**

| | Placebo beverage group (n = 22) | Test beverage group (n = 11) |
|---|---|---|
| Before consumption | 0 | 0 |
| After 12 weeks | -4 | -22 |

### Industrial applicability

The composition that enhances the production of adiponectin of the present invention contains a mannooligosaccharide having a degree of polymerization between 2 and 10 and can be produced by hydrolysis of mannan specifically. Chlorogenic acids can also be obtained from coffee beans. The composition that enhances adiponectin production of the present invention promises to enhance adiponectin production when consumed every day added to a food. The starting materials for the composition for enhancing adiponectin production of the present invention can use, for instance, coffee beans and coffee extract residue. That is, a composition that enhances adiponectin production is prepared from the residue of a coffee extract that would have been treated as a waste product in the past, and this composition can be consumed together with food and beverages, and the like. Therefore, the present invention is extremely useful in terms of enhancing health and using wasted resources.

## Claims

1. A pharmaceutical composition for enhancing adiponectin production, which contains a chlorogenic acid and a sugar having a degree of polymerization between 1 and 20 wherein mannose units account for at least 50% of the number of units.

2. The composition according to claim 1, wherein the sugar is a sugar having a degree of polymerization between 2 and 10 wherein mannose units account for at least 90% of the number of units.

3. The composition according to claim 1, wherein the sugar is a sugar having a degree of polymerization between 2 and 10 that is formed from mannose units alone.

4. An agent for enhancing adiponectin production according to any one of claims 1 through 3, wherein the sugar is β-1, 4-mannooligosaccharide.

5. The composition according to any one of claims 1 through 4, wherein the sugar is obtained by hydrolysis of mannan.

6. The composition according to claim 5, wherein the mannan is obtained from coffee beans and/or the residue of coffee extract.

7. A method for enhancing adiponectin production, the method comprising administering the composition according to claim 1, in the effective dose, to a patient requiring enhancement of adiponectin production.

8. A food, comprising a chlorogenic acid and a sugar having a degree of polymerization between 2 and 10 wherein mannose units account for at least 50% of the number of units.
